(19) Europäisches Patentamt
European Patent Office
Office européen des brevets

(11) Veröffentlichungsnummer: **0 047 328 B1**

# (12) EUROPÄISCHE PATENTSCHRIFT

(45) Veröffentlichungstag der Patentschrift: **09.05.84**

(51) Int. Cl.³: **C 07 D 403/12**, A 61 K 31/415

(21) Anmeldenummer: **80105327.3**

(22) Anmeldetag: **05.09.80**

(54) **1H- und 2H-Indazolderivate und diese enthaltendes Arzneimittel.**

(43) Veröffentlichungstag der Anmeldung: **17.03.82 Patentblatt 82/11**

(45) Bekanntmachung des Hinweises auf die Patenterteilung: **09.05.84 Patentblatt 84/19**

(84) Benannte Vertragsstaaten: **BE DE FR GB IT NL**

(56) Entgegenhaltungen:
**DE - A - 2 258 318**
**DE - A - 2 416 024**
**DE - A - 2 653 005**

(73) Patentinhaber: **SIEGFRIED AKTIENGESELLSCHAFT, CH-4800 Zofingen (CH)**

(72) Erfinder: **Molnar, Istvan, Dr., Eschenweg 10, CH-4800 Zofingen (CH)**
Erfinder: **Thiele, Kurt, Dr., Rebbergstrasse 47d, CH-4800 Zofingen (CH)**
Erfinder: **Geissmann, Felix, Brittnauerstrasse 28, CH-4800 Zofingen (CH)**
Erfinder: **Jahn, Ulrich, Dr., Kirchmoosstrasse 13, CH-4800 Zofingen (CH)**

(74) Vertreter: **Jaeger, Klaus, Dr.rer.nat. Dipl.-Chem. et al, Jaeger, Grams & Pontani Patentanwälte Bergstrasse 48 1/2, D-8035 München-Gauting (DE)**

**EP 0 047 328 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft 1H- und 2H-Indazolderivate der Formel

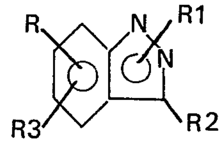

(I)

und deren Säureadditionssalze, wobei in der Formel I

R1 Wasserstoff, eine Alkylgruppe mit bis zu 6 Kohlenstoffatomen, eine Phenylalkylgruppe mit bis zu 6 Kohlenstoffatomen im Alkylteil oder eine Phenylgruppe bedeutet und an eines der beiden Stickstoffatome in 1- oder 2-Stellung gebunden ist, wobei die Phenylgruppe und der Phenylteil der Phenylalkylgruppe einfach oder mehrfach gleich oder verschieden mit einem Rest R4 substituiert sein können,

R2 und

R3 gleich oder verschieden voneinander sind und Wasserstoff, Halogen, die Hydroxylgruppe, eine Alkylgruppe mit bis zu 6 Kohlenstoffatomen eine Alkoxygruppe mit bis zu 6 Kohlenstoffatomen oder eine Alkylthiogruppe mit bis zu 6 Kohlenstoffatomen bedeuten, und

R4 und

R gleich oder verschieden voneinander ein Halogenatom oder eine der sekundären oder tertiären Aminogruppen

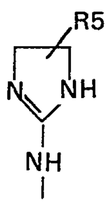
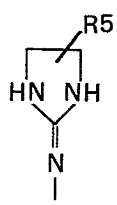
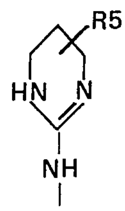
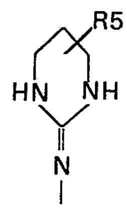

bedeuten, und zwar mit der Massgabe, dass zumindest einer der Reste R oder R4 eine der vorstehend genannten sekundären oder tertiären Aminogruppen ist und R für die 1H-Indazolderivate nur dann auch in 4- oder 7-Stellung eine der genannten sekundären oder tertiären Aminogruppen bedeuten kann, wenn der Rest R1 gleichzeitig eine Phenyl- oder Phenylalkylgruppe mit bis zu 6 Kohlenstoffatomen im Alkylteil ist, und wobei R5 Wasserstoff, die Hydroxylgruppe oder eine Alkyl- oder Alkoxygruppe mit jeweils bis zu 6 Kohlenstoffatomen ist.

Aus der DE-A1-2258318 und der DE-A1-2416.24 sind blutdrucksenkende (2-Imidazolin-2-yl-amino)-indazolderivate, die den erfindungsgemässen Verbindungen strukturell verwandt sind, bekannt. Diese bekannten Verbindungen entfalten ihre Wirkung ähnlich wie das Imidazolderivat Clonidin durch Beeinflussung adrenerger Rezeptoren im Zentralnervensystem.

Die erfindungsgemässen Verbindungen hingegen, insbesondere das 4-(2-Imidazolyl-amino)-2-methyl-indazol, üben vorwiegend periphere Wirkungen aus und unterscheiden sich in ihrem Wirkungsmechanismus gänzlich von den bekannten Verbindungen, da sie als partielle Antagonisten α-adrenerger Rezeptoren die Wirkung des Neurotransmitters Noradrenalin abschwächen, der durch Steuerung über sympatische Nervenimpulse freigesetzt wird und den Blutdruck regelt. Somit bilden die erfindungsgemässen Verbindungen eine wertvolle Alternative, wenn die bekannten Indazolderivate wegen ihrer Nebenwirkungen nicht zur Blutdruckregulierung verwendet werden können.

Die Erfindung betrifft weiterhin die Verwendung der eingangs genannten Substanzen als blutdrucksenkende Wirkstoffe zur Herstellung von Arzneimitteln für die Tier- und Humanmedizin.

Die Schreibweise des Indazolringsystems in der Formel I bedeutet, dass sowohl die 1H-Indazolderivate

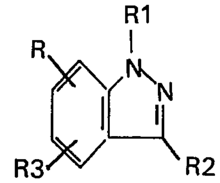

(Ia)

als auch die 2H-Indazolderivate

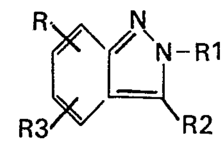

(Ib)

von der Formel I erfasst sein sollen.

Der Ausdruck «nieder» in Verbindung mit Alkylgruppen oder Alkylteilen in anderen Gruppierungen, speziell in den Alkoxy- und Phenylaralkylgruppen, bezeichnet Alkylgruppen bzw. Alkylteile mit bis zu 6 Kohlenstoffatomen, wobei diese Alkyle auch Cycloalkyle sein können. Vorzugsweise bezeichnet der Begriff «nieder» in diesem Zusammenhang jedoch gesättigte, verzweigte oder unverzweigte acyclische Kohlenwasserstoffreste mit insgesamt bis zu 4 Kohlenstoffatomen.

Zur Herstellung von Arzneimitteln finden selbstverständlich nur die pharmakologisch unbedenklichen Säureadditionssalze Verwendung, vorzugsweise die Hydrochloride und Nitrate.

Bezüglich des Restes R wird für die 2-Imidazolin-2-yl-derivate und die Imidazolidinderivate ebenso wie für die 2,4,5,6-Tetrahydropyrimidinderivate und die Hexahydropyrimidinderivate Tautomerie beobachtet.

Der Rest R ist vorzugsweise eine 2-Imidazolin-2-yl-aminogruppe (oder deren Tautomeres) oder ein Chloratom, wobei im zuletzt genannten Fall der R est R4 eine gegebenenfalls zusätzlich mit Chlor substituierte 2-Imidazolin-2-yl-phenyl- oder -benzylgruppe ist. Der Rest R1 ist vorzugsweise die Methylgruppe oder eine gegebenenfalls substituierte Benzylgruppe, während die Reste R2 und R3 vorzugsweise je ein Wasserstoffatom bedeuten.

Den folgenden Substanzen und ihren Säureadditionssalzen kommt eine besondere Bedeutung zu: 6-(2-Imidazolin-2-yl-amino)-1-methyl-1H-indazol, 4-(2-Imidazolin-2-yl-amino)-2-methyl-2H-indazol, 4-(2-Imidazolin-2-yl-amino)-2-benzyl-2H-indazol und 5-(2-Imidazolin-2-yl-amino)-1-methyl-1H-indazol.

Die Indazole der Formel I lassen sich nach den verschiedensten Verfahren herstellen.

Ein Verfahren zur Herstellung der Indazole der Formel I besteht darin, dass, wenn R kein Halogen ist, ein entsprechend substituiertes Indazol, das in der Stellung des Benzolringes, in der der Rest R eingeführt werden soll, eine Aminogruppe trägt, mit einer Verbindung der Formel

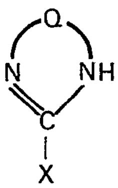 (IIa) oder 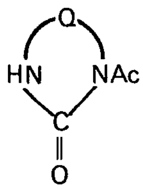 (IIb)

kondensiert wird, wobei X eine unter Kondensationsbedingungen mit Wasserstoff abspaltbare Gruppe, vorzugsweise ein Halogen, insbesondere Chlor, ist und Ac eine Acylgruppe bedeutet, vorzugsweise eine Alkylacylgruppe mit bis zu 6 Kohlenstoffatomen im Alkylteil oder eine substituierte oder unsubstituierte Phenylacylgruppe ist. Dabei bedeutet Q die Ethylen- oder Propylengruppe.

Wird als Ausgangssubstanz für diese Kondensation eine Verbindung der Formel IIa verwendet, so wird diese vorzugsweise in Form der freien Base in Gegenwart eines polaren oder nicht polaren Lösungsmittels eingesetzt.

Als Lösungsmittel dienen Alkohole, Äther oder chlorierte Kohlenwasserstoffe. Die Kondensation wird im Temperaturbereich zwischen Raumtemperatur und 150° C durchgeführt. Das als solches oder in Form seines Salzes ausfallende Produkt wird abgetrennt und aufgearbeitet.

Wenn der Substituent X in der Formel IIa eine Alkylthiogruppe oder die Nitroaminogruppe ist, so wird die Substanz der Formel IIa vorzugsweise als Salz eingesetzt und die Kondensation in Methanol oder Ethanol bei erhöhter Temperatur durchgeführt.

Wenn die Kondensation mit einer Substanz der Formel IIb vorgenommen wird, so kann insbesondere in einer Lösung oder Suspension des Amins, vorzugsweise in $POCl_3$, gearbeitet werden. Die Kondensation wird bei erhöhter Temperatur durchgeführt, und zwar bei Verwendung von $POCl_3$ bei Temperaturen bis zum Siedepunkt des $POCl_3$. Dabei wird die Acetylverbindung des Endprodukts erhalten, das dann in Essigsäure oder Methanol in die Verbindung der Formel I überführt wird.

Ein zweites Verfahren zur Herstellung der Substanzen der Formel I weist als charakteristisches Merkmal die Bildung des Imidazolin-, Imidazolidin- bzw. des Pyrimidinringes in situ auf. Bei diesen Verfahren wird ein Diamin der Formel $H_2N{-}Q{-}NH_2$, in der Q die Ethylen- oder Propylengruppe ist, als freie Base oder in Form eines Monosäureadditionssalzes mit einem entsprechend substituierten Indazol umgesetzt, das in der Stellung, in der der Rest R eingeführt werden soll, entweder eine Cyanaminogruppe, eine Gruppe

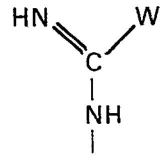

in der W eine unter Kondensationsbedingungen abspaltbare Alkylthiogruppe, Alkoxygruppe, Aminogruppe oder Nitroaminogruppe bedeutet, oder einen Rest $-N{=}C(Hal)_2$ trägt, wobei Hal ein Halogenatom bedeutet. Für die Herstellung der Imidazolin- und der Tetrahydropyrimidinderivate auf diesem Wege trägt das Indazolringsystem an der Stelle, an der der Substituent R eingeführt werden soll, vorzugsweise eine Isothiuroniumaminogruppe der Formel

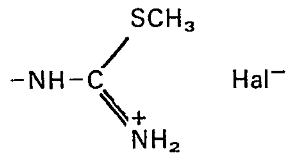

in der das Halogenid vorzugsweise Iodid ist. Insbesondere wenn dieser Substituent die Isothiuroniumiodidaminogruppe ist, wird die Ringschlusskondensation in einem inerten Lösungsmittel im Temperaturbereich von 40 bis 210° C durchgeführt, beispielsweise in Methanol oder Dioxan. Wenn der Substituent am Indazolringsystem die Cyanaminogruppe ist, erfolgt die Umsetzung in aliphatischen Alkoholen, Äther oder aliphatischen Kohlenwasserstoffen bei erhöhten Temperaturen im Bereich von 60 bis 220° C.

Das nach einer dieser Verfahrensvarianten erhaltene Reaktionsprodukt kann in üblicher Weise abgetrennt und durch Umkristallisieren gereinigt werden.

Nach einem dritten Verfahren kann der Rest R, sofern dieser eine der sekundären oder tertiären Aminogruppen bedeutet, schliesslich in der Weise eingeführt werden, dass an der für den Rest R vorgesehenen Stelle des Indazolringes zunächst die Gruppierung

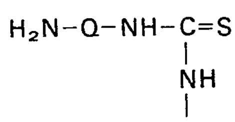

eingeführt wird, wobei in dieser Gruppierung der Schwefel auch durch ein Sauerstoffatom ersetzt sein kann und Q wiederum die Ethylen- oder Propylengruppe bedeutet, und an dieser Gruppe dann die Ringschlusskondensation vorgenommen wird. Die Reaktion wird vorzugsweise in einem polaren Lösungsmittel, beispielsweise Dimethylformamid, Methanol, Ethanol oder Wasser bei mässig erhöhten Temperaturen im Bereich von 30 bis 160° C in Gegenwart basischer Substanzen wie beispielsweise Alkalimetallhydroxiden vorgenommen.

Die für alle vorstehend genannten Verfahren zur Herstellung der Substanzen der Formel I benötigten Ausgangssubstanzen sind entweder als solche im Handel erhältlich oder nach in der Literatur beschriebenen Verfahren ohne weiteres herstellbar.

Die Erfindung ist im folgenden anhand von Ausführungsbeispielen näher erläutert.

*Beispiel 1*

*6-(2-Imidazolin-2-yl-amino)-1-methyl-indazolhydrochlorid*

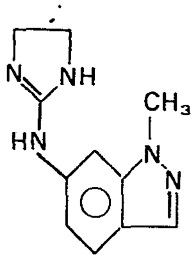

· HCl

10 g 2-Chlorimidazolinhydrogensulfat werden in 50 ml 2n NaOH gelöst. Die freie Base wird dann mit Methylenchlorid extrahiert. Das Lösungsmittel wird teilweise abdestilliert und der Rückstand mit Äther versetzt. Die erhaltene Lösung wird mit 4,5 g 6-Amino-1-methyl-indazol gelöst in 130 ml abs. THF versetzt. Das Gemisch wird 60 h bei Raumtemperatur gerührt. Dabei scheidet sich das Produkt in fester Form ab. Das ausgefallene Produkt wird abfiltriert und mit Äther gewaschen. Nach Umkristallisation aus absolutem Ethanol weist das Hydrochlorid des Produkts einen Schmelzpunkt von 247 bis 249° C auf.

*Elementaranalyse* für $C_{11}H_{14}N_5Cl$ (MG 251,7):

| | | | | |
|---|---|---|---|---|
| ber.: | C 52,49 | H 5,61 | N 27,82 | Cl 14,08% |
| ger.: | C 52,59 | H 5,70 | N 27,76 | Cl 14,16% |

*Beispiel 2*

*4-(2-Imidazolin-2-yl-amino)-2-methyl-indazolhydrochlorid*

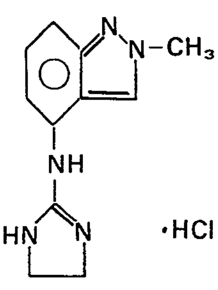

·HCl

4 g 4-Amino-2-methyl-indazol werden in 120 ml THF gelöst. Die Lösung wird mit einer Lösung von 2-Chlorimidazolinbase versetzt, die aus 10 g 2-Chlorimidazolinsulfat (hergestellt nach J. Heteroc. Chem. *11*, 258) mit 2n NaOH in Methylenchlorid erhalten wird.

Nach dreitägigem Stehen bei 20° C ist das Produkt Hydrochlorid vollständig ausgefallen. Das Produkt wird abfiltriert und aus Isopropanol umkristallisiert. Das umkristallisierte Hydrochlorid hat einen Schmelzpunkt von 270 bis 271° C und wird mit einer Ausbeute von 60% erhalten.

*Beispiel 3*

*4-(2-Imidazolin-2-yl-amino)-2-benzyl-indazol*

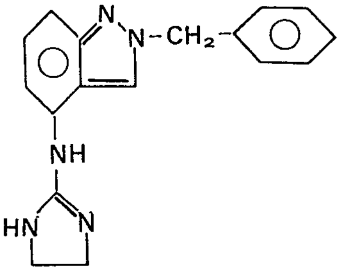

A) 6,9 g 4-Amino-2-benzyl-indazol werden in 100 ml absolutem THF gelöst. Die Lösung wird mit einer Lösung von 6,5 g Chlorimidazolin in 100 ml absolutem THF versetzt. Das Reaktionsprodukt wird anschliessend noch 38 h im verdunkelten geschlossenen Kolben bei Raumtemperatur gerührt. Dabei fällt das Reaktionsprodukt als weisser Niederschlag aus. Nach dem Dekantieren wird der Rückstand in 50 ml Wasser gelöst, mit 2n NaOH alkalisiert und dreimal mit je 100 ml Essigester extrahiert. Die Extrakte werden vereinigt und dreimal mit je 50 ml Wasser gewaschen. Anschliessend wird über Natriumsulfat getrocknet und auf ein Volumen von ca. 20 ml eingeengt. Dabei wird eine weisse kristalline Substanz erhalten, die abgetrennt und aus einem Gemisch aus Essigester und Methanol umkristallisiert wird. Es werden 1,3 g gereinigtes weisses kristallines Produkt mit einem Schmelzpunkt von 214 bis 216° C erhalten.

B) 15,2 g S-Methyl-N-(2-benzyl-indazol-4-yl)-isothiuroniumiodid werden in 155 ml Methanol gelöst und mit 3,2 ml Ethylendiamin versetzt. Das Gemisch wird 20 h unter Rückfluss auf Siedetemperatur gehalten. Das Reaktionsgemisch wird anschliessend eingeengt. Der erhaltene Rückstand wird in 50 ml heisser wässeriger 2n HCl gelöst. Nach dem Filtrieren wird die Lösung mit 2n NaOH versetzt und dreimal mit je 100 ml Chloroform extrahiert. Die vereinigten Extrakte werden mit Wasser gewaschen, über Natriumsulfat getrocknet und eingeengt. Der kristalline weisse Rückstand wird abgetrennt und aus einem Methanol-Essigester-Gemisch umkristallisiert. Dabei werden 5,06 g gereinigtes weisses kristallines Endprodukt mit einem Schmelzpunkt von 214 bis 216° C erhalten.

*Beispiel 4*

*5-(2-Imidazolin-2-yl-amino)-1-methyl-indazolhydrochlorid*

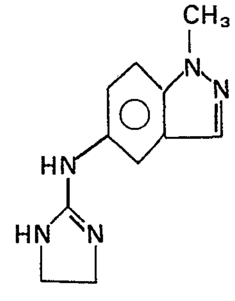

·HCl

5-Amino-1-methyl-indazol wird in absolutem THF gelöst und mit einer Lösung einer äquimolaren Menge von 2-Chlorimidazolin in Äther versetzt. Das Gemisch wird 48 h im verschlossenen und verdunkelten Kolben bei Raumtemperatur gerührt. Das ausgefallene Reaktionsprodukt wird abgetrennt und aus Ethanol umkristallisiert. Das gereinigte weisse kristalline Hydrochlorid hat einen Schmelzpunkt von 240 bis 241° C.

*Elementaranalyse* für $C_{11}H_{14}N_5Cl$ (MG 251,72):

| | | | | |
|---|---|---|---|---|
| ber.: | C 52,49 | H 5,61 | N 27,82 | Cl 14,08% |
| ger.: | C 52,58 | H 5,80 | N 28,0 | Cl 14,16% |

Analog zu den Verfahren der in Beispielen 1 bis 4 beschriebenen Art werden die folgenden Verbindungen hergestellt:

6-(2-Imidazolin-2-yl-amino)-2-methyl-indazoldihydrochlorid mit Smp. 217-219°;
7-(2-Imidazolin-2-yl-amino)-2-methyl-indazoldihydrochlorid mit Smp. 248-250°;
4-(2-Imidazolin-2-yl-amino)-1-benzyl-indazol mit Smp. 170-171°;
5-(2-Imidazolin-2-yl-amino)-1-benzyl-indazol mit Smp. 173-174°;
5-(2-Imidazolin-2-yl-amino)-1-(4-chlorbenzyl)-indazol mit Smp. 153-155°;
5-(2-Imidazolin-2-yl-amino)-2-benzyl-indazol mit Smp. 186-189°;
7-(2-Imidazolin-2-yl-amino)-1-benzyl-indazol mit Smp. 177-179°;
7-(2-Imidazolin-2-yl-amino)-2-benzyl-indazol mit Smp. 180-182°;
5-Chlor-1-(2-(2-imidazolin-2-yl-amino)-phenyl)-indazol mit Smp. 186-188°;
5-Chlor-1-(3,5-dichlor-4-(2-imidazolin-2-yl-amino)-phenyl)-indazolhydrochlorid mit Smp. 333-334°;
5-Chlor-1-(3,5-dichlor-2-(2-imidazolin-2-yl-amino)-phenyl)-indazol mit Smp. 241-243°; und
5-Chlor-1-(3-(2-imidazolin-2-yl-amino)-benzyl)-indazol mit Smp. 216-218°.

## Patentansprüche

1. 1H- und 2H-Indazolderivate der Formel

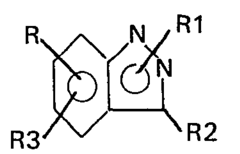

(I)

und deren Säureadditionssalze, wobei in der Formel I

R1 Wasserstoff, eine Alkylgruppe mit bis zu 6 Kohlenstoffatomen, eine Phenylalkylgruppe mit bis zu 6 Kohlenstoffatomen im Alkylteil oder eine Phenylgruppe bedeutet und an eines der beiden Stickstoffatome in 1- oder 2-Stellung gebunden ist, wobei die Phenylgruppe und der Phenylteil der Phenylalkylgruppe einfach oder mehrfach gleich oder verschieden mit einem Rest R4 substituiert sein können,

R2 und

R3 gleich oder verschieden voneinander sind und Wasserstoff, Halogen, die Hydroxylgruppe, eine Alkylgruppe mit bis zu 6 Kohlenstoffatomen eine Alkoxygruppe mit bis zu 6 Kohlenstoffatomen oder eine Alkylthiogruppe mit bis zu 6 Kohlenstoffatomen bedeuten, und

R4 und

R gleich oder verschieden voneinander ein Halogenatom oder eine der sekundären oder tertiären Aminogruppen

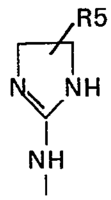 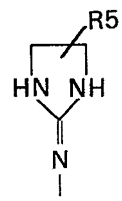 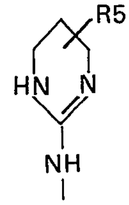 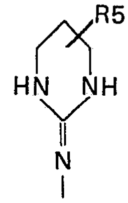

bedeuten, und zwar mit der Massgabe, dass zumindest einer der Reste R oder R4 eine der vorstehend genannten sekundären oder tertiären Aminogruppen ist und R für die 1H-Indazolderivate nur dann auch in 4- oder 7-Stellung eine der genannten sekundären oder tertiären Aminogruppen bedeuten kann, wenn der Rest R1 gleichzeitig eine Phenyl- oder Phenylalkylgruppe mit bis zu 6 Kohlenstoffatomen im Alkylteil ist, und wobei R5 Wasserstoff, die Hydroxylgruppe oder eine Alkyl- oder Alkoxygruppe mit jeweils bis zu 6 Kohlenstoffatomen ist.

2. Arzneimittel, insbesondere blutdrucksenkendes Arzneimittel, enthaltend mindestens eines der 1H- oder 2H-Indazolderivate nach Anspruch 1 und/oder deren pharmazeutisch unbedenklichen Salze.

## Claims

1. 1H and 2H indazole derivatives of the formula

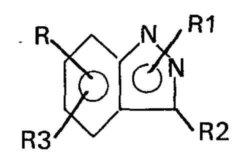

(I)

and acid addition salts thereof, in formula I

R1 representing hydrogen, an alkyl group containing up to 6 carbon atoms, a phenylalkyl group containing up to 6 carbon atoms in the alkyl moiety or a phenyl group and being connected to one of the two nitrogen atoms in 1 or 2 position, the phenyl group and the phenyl moiety of the phenylalkyl group being able to be substituted once or multiply, equally or non-equally by a radical R4,

R2 and

R3 being equal or different from each other and representing hydrogen, halogen, the hydroxyl group, an alkyl group containing up to 6 carbon atoms, an alkoxy group containing up to 6 carbon atoms or an alkylthio group containing up to 6 carbon atoms, and

R4 and

R being equal or different from each other and representing a halogen atom or one of the secondary or tertiary amino groups

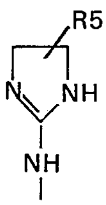
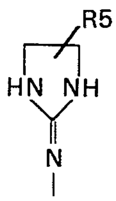
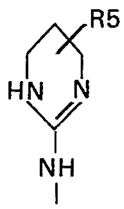
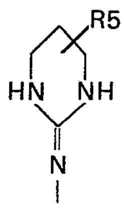

with the proviso that at least one of the radicals R or R4 is one of the above mentioned secondary or tertiary amino groups and R for the 1H indazole derivatives can only represent also in 4 or 7 position one of the mentioned secondary or tertiary amino groups, if the radical R1 is simultaneously a phenyl group or a phenylalkyl group containing up to 6 carbon atoms in the alkyl moiety and R5 being hydrogen, the hydroxyl group or an alkyl or alkoxy group, each containing up to 6 carbon atoms.

2. Medicine, especially a blood-pressure-lowering medicine, containing at least one of the 1H or 2H indazole derivatives according to claim 1 and/or the pharmaceutically inobjectionable salts thereof.

## Revendications

1. Dérivés de 1H- et de 2H-indazole de formule

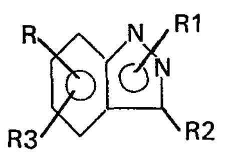

(I)

et leurs sels d'addition acide, les symboles dans la formule I ayant la signification suivante:

R1 est de l'hydrogène, un groupe alkyle ayant jusqu'à 6 atomes de carbone, un groupe phénylalkyle ayant jusqu'à 6 atomes de carbone dans la partie alkyle ou un groupe phényle, et R1 est lié à l'un des deux atomes d'azote en position 1 ou 2, le groupe phényle et la partie phényle du groupe phénylalkyle pouvant être substitués une ou plusieurs fois de façon identique ou différente par un reste R4,

R2 et

R3 sont identiques ou différents et sont de l'hydrogène, de l'halogène, le groupe hydroxyle, un groupe alkyle avant jusqu'à 6 atomes de carbone, un groupe alcoxy ayant jusqu'à 6 atomes de carbone ou un groupe alkylthio ayant jusqu'à 6 atomes de carbone, et

R4 et

R peuvent être identiques ou différents et sont un atome d'halogène ou un des groupes amino secondaires ou tertiaires suivants:

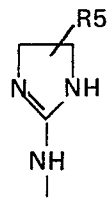
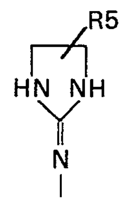
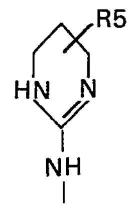
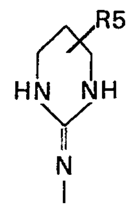

avec la restriction toutefois qu'au moins un des restes R ou R4 soit un des groupes amino secondaires ou tertiaires sus-indiqués et que R dans les dérivés de 1H-indazole ne puisse être, également en position 4 ou 7, un des groupes amino secondaires ou tertiaires que dans le cas où le reste R1 est simultanément un groupe phényle ou phénylalkyle ayant dans la partie alkyle jusqu'à 6 atomes de carbone, R5 étant de l'hydrogène, le groupe hydroxyle ou un groupe alkyle ou alcoxy ayant chacun jusqu'à 6 atomes de carbone.

2. Médicaments, en particulier des médicaments hypoglycémiants, contenant au moins un des dérivés de 1H- ou 2H-indazole selon la revendication 1 et/ou leurs sels pharmacologiquement insoupçonnables.